# EUROPEAN PATENT SPECIFICATION

(11) **EP 2 464 350 B1**
(45) Date of publication and mention of the grant of the patent: **24.08.2016**
(21) Application number: 10815735.5
(22) Date of filing: 02.09.2010
(51) Int. Cl.: A61K 31/05, A61K 31/07, A61K 31/122, A61K 33/30, A61K 45/06, G06Q 30/02, A61K 31/4045

(54) **COMPOSITIONS AND METHODS FOR ENHANCING COGNITIVE AND RELATED FUNCTIONS IN ANIMALS**
ZUSAMMENSETZUNGEN UND VERFAHREN ZUR INTENSIVIERUNG KOGNITIVER UND VERWANDTER FUNKTIONEN BEI TIEREN
COMPOSITIONS ET PROCÉDÉS DESTINÉS À AMÉLIORER LES FONCTIONS COGNITIVES ET ASSOCIÉES CHEZ LES ANIMAUX

(30) Priority: 11.09.2009 US 276402 P
(43) Date of publication of application: 20.06.2012
(73) Proprietor: Nestec S.A., 1800 Vevey (CH)
(72) Inventor: ZANGHI, Brian, Michael, Gowanda, NY 14070 (US); RAMADAN, Ziad, Saad, Manchester, MO 63021 (US)
(74) Representative: Rupp, Christian
(86) International application number: PCT/US2010/002440
(87) International publication number: WO 2011/031304

(56) References cited:
- EP-A1- 1 283 038
- WO-A1-02/30412
- WO-A1-99/43329
- WO-A1-2009/088433
- US-A1- 2004 001 817
- US-A1- 2004 162 329
- US-A1- 2006 039 954
- US-A1- 2008 167 363

## Description

### CROSS REFERENCE TO RELATED APPLICATIONS

This application claims priority to U.S. Provisional Application Serial No. 61/276402 filed September 11, 2009.

### BACKGROUND OF THE INVENTION

### Field of the Invention

The invention relates generally to compositions and methods for enhancing cognitive function and particularly to compositions comprising melatonin and one or more carotenoids and their use for enhancing cognitive and related functions in companion animals animals.

### Description of Related Art

Aged or aging animals frequently suffer some degree of cognitive impairment. Changes such as a decline in cognitive function that progresses with age and age-related changes in brain morphology and cerebrovascular function are commonly observed in aging animals, e.g., brain aging. Age-related or age-associated cognitive impairment may manifest itself in many ways, e.g., short-term memory loss, diminished capacity to learn, diminished rate of learning, diminished attention, and/or dementia. In some cases, a specific etiology of such cognitive decline is unknown. In other cases, cognitive impairment results from the onset or progression of recognized diseases, disorders, or syndromes such as Alzheimer's disease (AD). However, it is known that age-associated cognitive decline is distinct from and can occur independently of AD.

Animal models of cognitive impairment greatly facilitate the study of such conditions including their physiology, neurology, anatomy, and pathology. Dogs are useful model animals that demonstrate age-associated cognitive decline in learning and memory that varies depending on the function of the cognitive task (Adams B *et al.,* 2000a; Chan ADF *et al.,* 2002; Su M-Y *et al.,* 1998; and, Tapp PD *et al.,* 2003). While the study of such decline in dogs is useful in its own right because of their role as companion animals, the fact that the observed decline mirrors age-related cognitive declines seen in humans (Adams B *et al.* 2000b) makes the studies even more valuable. Aged dogs develop neuropathology that is related to that seen in both successfully aging humans and patients with AD, such as beta amyloid protein (Cotman CW and Berchtold, 2002; and Cummings BJ *et al.,* 1996). However, dogs do not demonstrate every hallmark of AD, in particular, tau-containing neurofibrillar tangles (Dimakopoulos AC *et al.,* 2002) have not been observed. Therefore, the condition in dogs is distinct and referred to as Canine Cognitive Dysfunction Syndrome (CCDS).

Both healthy dogs and unhealthy dogs such as those diagnosed with CCDS may present clinically with progressive cognitive impairment and neuropathological changes (London ED *et al.,* 1983). In addition, aging dogs and those diagnosed with CCDS exhibit various cognitive impaired related behavioral disorders. For example, the animals may not respond to their name or familiar commands, get lost or confused even in familiar surroundings, no longer greet or respond to their owners or visitors, exhibit diminished daytime activity, walk in circles, shun affection, and lose bladder or bowel control.

WO 02/30412 A2 is directed to compositions comprising tryptamines, carotenoids and tocotrienol species providing for antioxidant activity. WO 02/30412 A2 is silent on the addition of zinc or a combination of melatonin, one or morc carotenoids and zinc.

EP 1 283 038 A1 discloses a composition comprising astaxanthin and melatonin. EP 1 283 038 A1 does not mention the use of zinc or an enhancement of cognitive function. EP 1 283 038 A1 is also silent on a combination of melatonin, one or more carotenoids and zinc.

WO 99/43329 A1 discloses a composition comprising a) at least one hormone; b) at least one amino acid; c) at least one enzyme and/or vitamin; and d) at least one mineral. WO 99/43329 A1 is described to be effective in treating conditions such as cardiovascular diseases, auto-immune diseases, Parkinson's disease, particularly cardiovascular diseases. WO 99/43329 A1 is silent about the enhancement of cognitive function or the prevention, reduction, or delay of a decline in cognitive and related functions, respectively.

US 2004/0001817 A1 discloses an anti-aging nutritional supplement composition which comprises vitamins, minerals, an inflammatory process support, a blood sugar/insulin support, a botanical antioxidants, a methylating factor, a DNA repair agent, a fat metabolizer, an absorption enhancer, a brain function support, whole foods, a cellular energizer, a nucleotide precursor, amino acids, a fatty acid complex, and digestive enzymes. US 2004/0001817 A1 fails to disclose the specific combination of melatonin, carotenoids and zinc in an amount effective for enhancing cognitive function.

US 2008/0167363 relates to methods for modulating neurogenesis comprising administering a melatoninergic agent, optionally in combination with one or more neurogenic agents. US 2008/0167363 is silent about zinc. WO 2009/088433 A1 discloses compositions suitable for enhancing cognitive and related functions in an animal comprising one or more unsaturated fatty acids, one or more nitric oxide releasing compounds in an amount effective for enhancing cognitive function in an animal. WO 2009/088433 A1 is silent on a combination of melatonin, one or more carotenoids and zinc.

Though advances have been made, there remains a need to develop compositions and methods useful for enhancing cognitive and related functions, particularly in aging humans and other animals. Such compositions and methods would be useful to promote the health and wellness of and improve the overall quality of life for such animals and their caregivers. For companion animals, these compositions and methods would lead to improved owner satisfaction and would improve the owner-companion animal bond.

### SUMMARY OF THE INVENTION

It is, therefore, an object of the present invention to provide compositions useful for enhancing cognitive function in an animal.

Described herein are furthermore methods for reducing or preventing a decline of social interaction, reducing or preventing age-related behavioral changes, increasing trainability, improving attention, maintaining optimal brain function, facilitating learning and memory, reducing memory loss, and preventing or treating cognitive decline caused by dementia in an animal.

Also described herein are articles of manufacture in the form of kits that contain one or more of the compounds useful to produce the composition of the present invention in combination with foods or other ingredients and devices useful for enhancing cognitive function in an animal.

It is also described to provide a package comprising a composition of the present invention and a label affixed to the package indicating the contents of the package and/or the benefits of administering the composition to an animal.

The object underlying the present invention is achieved using novel compositions for enhancing cognitive function according to the attached claims. The compositions comprise melatonin and one or more carotenoids, and zinc. The methods described herein show administering of the compositions to a companion animal in an amount effective for enhancing cognitive function.

Other and further objects, features, and advantages of the present invention will be readily apparent to those skilled in the art.

### DETAILED DESCRIPTION OF THE INVENTION

### Definitions

The term "animal" means any animal that can benefit from compositions and methods useful for enhancing cognitive function, *e.g.,* a human, avian, bovine, canine, equine, feline, hicrine, lupine, murine, ovine, and porcine animal.

The term "companion animal" means any domesticated animal such as cats, dogs, rabbits, guinea pigs, ferrets, hamsters, mice, gerbils, horses, cows, goats, sheep, donkeys, pigs, and the like.

The term "effective amount" means an amount of a composition, compound, medicament, or other material that is effective to achieve particular desired results, e.g., enhancing cognitive function, reducing or preventing a decline of social interaction, reducing or preventing age-related behavioral changes, increasing trainability, improving attention, maintaining optimal brain function, facilitating learning and memory, reducing memory loss, preventing or treating cognitive decline caused by dementia, maintaining mental clarity and alertness, promoting health and wellness, improving quality of life, and extending the prime.

The term "cognitive function" means the special, normal, or proper physiologic activity of the brain, including one or more of the following: mental stability, memory/recall abilities, problem solving abilities, reasoning abilities, thinking abilities, judging abilities, ability to discriminate or make choices, capacity for learning, ease of learning, perception, intuition, attention, and awareness.

The term "enhancing cognitive function" means one or more of increasing cognitive and related functions in an animal or preventing, reducing, or delaying a decline in cognitive and related functions in an animal, e.g., increasing cognitive function, reducing or preventing a decline of social interaction, reducing or preventing age-related behavioral changes, increasing trainability, improving attention, maintaining optimal brain function, facilitating learning and memory, reducing memory loss, preventing or treating cognitive decline caused by dementia, maintaining mental clarity and alertness, promoting health and wellness, improving quality of life, and extending the prime.

The term "behavior" means anything that an animal does in response or reaction to a given stimulation or set of conditions.

The term "decline" of any of the foregoing categories or specific types of functions in an individual (phenotypes) is generally the opposite of an improvement or enhancement in the function. An "effective amount" of a composition may be an amount required to prevent decline altogether or to substantially prevent decline ("prevent" decline), to reduce the extent or rate of decline ("reduce" decline) over any time course or at any time point, or delay the onset, extent, or progression of a decline ("delay" a decline). Prevention, reduction, or delay of decline is frequently a more useful comparative basis when working with non-diseased aging animals (e.g., "healthy aging animals"). Prevention, reduction, and delay of decline can be considered relative to a control or cohort that does not receive the treatment, e.g., a composition of interest. Prevention, reduction, or delay of either the onset of a detrimental quality or condition, or of the rate of decline in a particular function can be measured and considered on an individual basis, or in some embodiments on a population basis. The net effect of preventing, reducing, or delaying decline is to have less decrease in cognitive or behavioral function per unit time, or at a given end point. In other words, ideally, for an individual or in a population, cognitive and behavioral function is maintained at the highest possible level for the longest possible time. Thus, it is not required that there be a net increase in cognitive or behavioral function for any embodiment. For purposes herein, an individual can be compared to a control individual, group, or population. A population can likewise be compared to an actual individual, to normalized measurements for an individual, or to a group or population, as is useful.

The term "aging" means being of advanced age such that the animal has exceeded 50% of the average lifespan for its particular species and/or breed within a species. For example, if the average lifespan for a given breed of dog is 10 years, then a dog within that breed greater than 5 years old would be considered "aging" for purposes herein. A determination of lifespan may be based on actuarial tables, calculations, estimates, or the like.

The term "food product formulated for human consumption" means any composition specifically intended for ingestion by a human.

The term "pet food" or "pet food composition" means a composition intended for consumption by animals, preferably by companion animals.

The term "complete and nutritionally balanced pet food" mean a pet food that contains all known required nutrients for the intended recipient or consumer of the food, in appropriate amounts and proportions, based for example on recommendations of recognized authorities in the field of companion animal nutrition. Such foods are therefore capable of serving as a sole source of dietary intake to maintain life or promote production, without the addition of supplemental nutritional sources. Nutritionally balanced pet food compositions are widely known and used in the art. The term includes any food, feed, snack, food supplement, treat, meal substitute, or meal replacement, whether intended for a human or another animal. Animal food includes food or feed intended for any domesticated or wild species. In preferred embodiments, a food for an animal represents a nutritionally complete food composition, e.g., a pelleted, extruded, or dry food. Examples of such animal foods include extruded pet foods, such as foods for dogs and cats.

The term "dietary supplement" means a product that is intended to be ingested in addition to the normal animal diet. Dietary supplements may be in any form, e.g., solid, liquid, gel, tablets, capsules, powder, and the like. Preferably they are provided in convenient dosage forms. In some embodiments they are provided in bulk consumer packages such as bulk powders, liquids, gels, or oils. In other embodiments, supplements are provided in bulk quantities to be included in other food items such as snacks, treats, supplement bars, beverages and the like.

The term "long-term administration" means periods of repeated administration or consumption in excess of one month. Periods of longer than two, three, or four months are preferred for certain embodiments. Also preferred are more extended periods that include longer than 5, 6, 7, 8, 9, or 10 months. Periods in excess of 11 months or 1 year are also preferred. Longer term use extending over 1, 2, 3, or more years are included in the invention. For certain aging animals, the animal will continue consuming on a regular basis for the remainder of its life.

The term "regular basis" means at least monthly dosing with the compositions or consumption of the compositions, more preferably weekly dosing. More frequent dosing or consumption, such as twice, three, or seven times weekly, is preferred in certain embodiments. Still more preferred are regimens that comprise at least once daily consumption. The skilled artisan will appreciate that the blood level of a compound or certain metabolites of that compound or which result after the consumption of that compound, may be a useful tool for assessing or determining dosing frequency. A frequency, regardless of whether expressly exemplified herein, that allows maintenance of a desired blood level of the measured compound within acceptable ranges is useful herein. The skilled artisan will appreciate that dosing frequency will be a function of the composition that is being consumed or administered, and some compositions may require more or less frequent administration to maintain a desired blood level of the measured compound.

The term "cognitive drugs" means any compound, composition, or drug useful for affecting cognitive function, e.g., monoamine oxidase B inhibitors such as selegiline; vasodilators such as nicerogoline and vinpocetine; phosphatidylserine; propentofyline; anticholinesterases (cholinesterase inhibitors) such as tacrine, galantamine, rivastigmine, vinpocetine, donepezil (ARICEPT® (donepezil hydrochloride)), metrifonate, and physostigmine; lecithin; choline cholinomimetics such as milameline and xanomeline; ionotropic N-methyl-D-aspartate (NMDA) receptor antagonists such as memantine; anti-inflammatory drugs such as prednisolone, diclofenac, indomethacin, propentofyline, naproxen, rofecoxin, ibruprofen and suldinac; metal chelating agents such as cliquinol; *Ginkgo biloba;* bisphosophonates; selective oestrogen receptor modulators such as raloxifene and estrogen; a phytoestrogen; beta and gamma secretase inhibitors; cholesterol-lowering drugs such as statins; calcitonin; risedronate; alendronate; and combinations thereof. Cognitive drugs also include compositions known to affect cognitive function in animals such as those disclosed in published patent applications WO2009/045481, WO2010/014245, WO2007/070701, WO2007/041418, and WO2009/088433,

The term "in conjunction" means that a composition comprising melatonin, carotenoids, zinc, a food composition, cognitive drug, or other compound or composition of the present invention are administered to an animal (1) together in a food composition or (2) separately at the same or different frequency using the same or different administration routes at about the same time or periodically. "Periodically" means that the agent is administered on a dosage schedule acceptable for a specific agent and that the food is fed to an animal routinely as appropriate for the particular animal. "About the same time" generally means that the food and agent are administered at the same time or within about 72 hours of each other. "In conjunction" specifically includes administration schemes wherein a cognitive drug is administered for a prescribed period and the compositions comprising melatonin, carotenoids, and zinc are administered indefinitely.

The term "individual" when referring to an animal means an individual animal of any species or kind.

The term "single package" means that the components of a kit are physically associated, in or with one or more containers, and considered a unit for manufacture, distribution, sale, or use. Containers include, but are not limited to, bags, boxes or cartons, bottles, packages of any type or design or material, over-wrap, shrink-wrap, affixed components (e.g., stapled, adhered, or the like), or combinations of any of the foregoing. For example, a single package kit may provide containers of individual compositions and/or food compositions physically associated such that they are considered a unit for manufacture, distribution, sale, or use.

The term "virtual package" means that the components of a kit are associated by directions on one or more physical or virtual kit components instructing the user how to obtain the other components, e.g., in a bag or other container containing one component and directions instructing the user to go to a website, contact a recorded message or a fax-back service, view a visual message, or contact a caregiver or instructor to obtain, for example, instructions on how to use the kit, or safety or technical information about one or more components of a kit. Examples of information that can be provided as part of a virtual kit include instructions for use; safety information such as material safety data sheets; poison control information; information on potential adverse reactions; clinical study results; dietary information such as food composition or caloric composition; general information on cognitive or behavioral function; diseases that effect cognitive or behavioral function; treating cognitive or behavioral function; or general information on treatment or preservation of cognitive or behavioral function; self-help relating to cognitive or behavioral function; caregiver information for those caring for animals with cognitive or behavioral function challenges; and use, benefits, and potential side-effects or counter-indications for cognitive drugs.

The term "health and wellness of an animal" means the complete physical, mental, and social well being of the animal, not merely the absence of disease or infirmity.

The term "quality of life" means the ability to enjoy normal life activities.

The term "extending the prime" means extending the number of years an animal lives a healthy life and not just extending the number of years an animal lives, *e.g.,* an animal would be healthy in the prime of its life for a relatively longer time.

All percentages expressed herein are by weight of the composition on a dry matter basis unless specifically stated otherwise. The skilled artisan will appreciate that the term "dry matter basis" means that an ingredient's concentration or percentage in a composition is measured or determined after any free moisture in the composition has been removed.

As used throughout, ranges are used herein in shorthand, so as to avoid having to set out at length and describe each and every value within the range. Any appropriate value within the range can be selected, where appropriate, as the upper value, lower value, or the terminus of the range.

As used herein and in the appended claims, the singular form of a word includes the plural, and vice versa, unless the context clearly dictates otherwise. Thus, the references "a", "an", and "the" are generally inclusive of the plurals of the respective terms. For example, reference to "a puppy", "a method", or "a food" includes a plurality of such "puppies", "methods", or "foods". Reference herein, for example to "an antioxidant" includes a plurality of such antioxidants, whereas reference to "pieces" includes a single piece. Similarly, the words "comprise", "comprises", and "comprising" are to be interpreted inclusively rather than exclusively. Likewise the terms "include", "including" and "or" should all be construed to be inclusive, unless such a construction is clearly prohibited from the context. Where used herein the term "examples," particularly when followed by a listing of terms is merely exemplary and illustrative, and should not be deemed to be exclusive or comprehensive.

The methods and compositions and other advances disclosed here are not limited to particular methodology, protocols, and reagents described herein because, as the skilled artisan will appreciate, they may vary. Further, the terminology used herein is for the purpose of describing particular embodiments only, and is not intended to, and does not, limit the scope of that which is disclosed or claimed.

Unless defined otherwise, all technical and scientific terms, terms of art, and acronyms used herein have the meanings commonly understood by one of ordinary skill in the art in the field(s) of the invention, or in the field(s) where the term is used. Although any compositions, methods, articles of manufacture, or other means or materials similar or equivalent to those described herein can be used in the practice of the present invention, the preferred compositions, methods, articles of manufacture, or other means or materials are described herein.

Concerning all patents, patent applications, publications, technical and/or scholarly articles, and other references cited or referred to herein the discussion of those references is intended merely to summarize the assertions made therein. No admission is made that any such patents, patent applications, publications or references, or any portion thereof, are relevant, material, or prior art. The right to challenge the accuracy and pertinence of any assertion of such patents, patent applications, publications, and other references as relevant, material, or prior art is specifically reserved. Full citations for publications not cited fully within the specification are set forth at the end of the specification.

### The Invention

The invention provides compositions suitable for enhancing cognitive function in a companion animal according to the attached claims. The compositions comprise melatonin and one or more carotenoids in an amount effective for enhancing cognitive function in the animal. The compositions further comprise zinc in an amount effective for enhancing cognitive function in the animal.

The invention is based upon the discovery that compositions comprising melatonin and carotenoids, and zinc, are effective for enhancing cognitive function in animals. The compositions enhance cognitive function in animals of all ages, e.g., juvenile, adult, senior, or geriatric animals. The compositions enhance cognitive function in healthy animals or animals that are susceptible to or suffering from a decline in cognitive function brought about by the aging process or by injury or disease. The compositions are particularly effective for reducing or delaying the effects of age-related and disease-related cognitive decline in humans and companion animals, particularly dogs and cats. The compositions are also useful for enhancing cognitive function when cognitive decline is caused by changes in brain function, particularly brain aging or brain damage from injury or disease such as strokes or dementia.

The melatonin is any melatonin suitable for administration to an animal. Melatonin is obtained from any suitable source, synthetic or natural. A preferred source of melatonin is synthetic melatonin, e.g., in tablet, capsule, and sublingual tablet forms. Other sources include melatonin from natural sources such as algae, fungi, and plants, e.g., seeds and walnuts. Melatonin may be derived from a blend of melatonin sources, whether natural or synthetic.

The carotenoids are any carotenoids suitable for administration to an animal. Carotenoids are obtained from any suitable source, synthetic or natural. Preferred sources of carotenoids are natural sources such as chloroplasts, chromoplasts, algae, fungus, and bacteria that contain carotenoids. Other preferred sources include seafood such as salmon, trout, red sea bream, shrimp and lobster and birds such as flamingo and quail. A particularly preferred source is the algae *Haematococcus pluvialis.* The carotenoids may also be synthetic and as such may be produced using any suitable starting materials and any means available in the art. The carotenoids may comprise a blend of any one or more carotenoids from any one or more such sources, whether natural or synthetic. In preferred embodiments, the carotenoids are xanthophylls. In some embodiments, the xanthophylls are selected from the group consisting of astaxanthin, lutein, zeaxanthin, neoxanthin, violaxanthin, α-cryptoxanthin, and β-cryptoxanthin. In a preferred embodiment, the carotenoid or xanthophyll is astaxanthin.

The zinc is any zinc suitable for administration to an animal. Zinc can be obtained from any source suitable for administration to an animal, e.g., minerals or plants. The zinc can be obtained from foods such as meats, vegetables, eggs, herbs, and other food sources known to skilled artisans. Zinc can also be obtained from minerals and zinc compounds suitable for consumption by animals, e.g., zinc acetate, zinc chloride, zinc sulfate, and zinc gluconate.

The compositions comprise melatonin and one or more carotenoids, and zinc, in an amount effective for enhancing cognitive function. The compositions comprise from 0.1 to 80 mg/kg melatonin and from 0.01 to 20 mg/kg carotenoids, e.g., astaxanthin. The composition may comprise from 0.1 to 400 mg/kg zinc. In various embodiments, the compositions comprise melatoninfrom 0.1 to 80 mg/kg; and preferably from 0.02 to 10 mg/kg carotenoids. In one embodiment, the compositions comprise melatonin, one or more carotenoids, and zinc in such amounts.

In various embodiments, the compositions comprise melatonin and carotenoids, and zinc, in amounts effective for one or more of reducing or preventing a decline of social interaction, reducing or preventing age-related behavioral changes, increasing trainability, improving attention, maintaining optimal brain function, facilitating learning and memory, reducing memory loss, preventing or treating cognitive decline caused by dementia, maintaining mental clarity and alertness, promoting health and wellness, improving quality of life, and extending the prime.

In other embodiments, the composition is formulated to provide melatonin in amounts of from 0.1 to 30 mg per day and carotenoids in amounts of from 0.2 to 20 mg per day to an animal. In another, composition is formulated to further provide zinc in amounts of from 10 to 100 mg per day to an animal.

In various embodiments, the compositions are produced by combining different sources or forms of melatonin, carotenoids, and zinc, e.g., melatonin produced synthetically and one or more carotenoids contained in or extracted from a natural source such as a mineral, plant, or animal. In one embodiment, the melatonin is from a natural source and the carotenoids are from a different or the same natural source. In another, the melatonin is from a synthetic source, the carotenoids are from a natural source, and the zinc is from a mineral source. In another, the melatonin is from a synthetic source, the carotenoids are from a natural source, and the zinc is from a natural source. In a preferred embodiment, the melatonin is from a synthetic source, the carotenoid is from algae (*e.g., H. pluvialis*), and the zinc is from a mineral source. In a further embodiment, the melatonin is from algae, the carotenoid is from algae, and the zinc is from a plant source. In another, the melatonin is from a plant source (phytomelatonin), the carotenoid is from algae (*e.g., H. pluvialis*), and the zinc is from a food source. Many such combinations are possible and will be known to or discernable by skilled artisan.

In another embodiment, the compositions further comprise one or more cognitive drugs in an amount effective for enhancing cognitive function. The skilled artisan can determine the amount of cognitive drug to be added to the composition based upon the recommended dosage for the drug given by its manufacturer or upon the animal's weight, species, age, health status, and the like.

In other embodiments, the compositions further comprise prebiotics, probiotics, or a combination thereof. Probiotics are live microorganisms that have a beneficial effect in the prevention and treatment of specific medical conditions when ingested. Probiotics are believed to exert biological effects through a phenomenon known as colonization resistance. The probiotics facilitate a process whereby the indigenous anaerobic flora limits the concentration of potentially harmful (mostly aerobic) bacteria in the digestive tract. Other modes of action, such as supplying enzymes or influencing enzyme activity in the gastrointestinal tract, may also account for some of the other functions that have been attributed to probiotics. Prebiotics are nondigestible food ingredients that beneficially affect host health by selectively stimulating the growth and/or activity of bacteria in the colon. Prebiotics include fructooligosaccharides (FOS), xylooligosaccharides (XOS), galactooligosaccharides (GOS), and mannooligosaccharides (typically for non-human foods such as petfoods). The prebiotic, fructooligosaccharide (FOS) is found naturally in many foods such as wheat, onions, bananas, honey, garlic, and leeks. FOS can also be isolated from chicory root or synthesized enzymatically from sucrose. FOS fermentation in the colon results in a large number of physiologic effects including increasing the numbers of bifidobacteria in the colon, increasing calcium absorption, increasing fecal weight, shortening of gastrointestinal transit time, and possibly lowering blood lipid levels. The increase in bifidobacteria has been assumed to benefit human health by producing compounds to inhibit potential pathogens, by reducing blood ammonia levels, and by producing vitamins and digestive enzymes. Probiotic bacteria such as Lactobacilli or Bifidobacteria are believed to positively affect the immune response by improving the intestinal microbial balance leading to enhanced antibody production and phagocytic (devouring or killing) activity of white blood cells. *Bifidobacterium lactis* could be an effective probiotic dietary supplement for enhancing some aspects of cellular immunity in the elderly. Probiotics enhance systemic cellular immune responses and may be useful as a dietary supplement to boost natural immunity in otherwise healthy adults. Probiotics include many types of bacteria but generally are selected from four genera of bacteria: *Lactobacilllus acidophillus, Bifidobacteria, Lactococcus,* and *Pediococcus.* Beneficial species include *Enterococcus* and *Saccharomyces* species, *e.g., Enterococcus faecium* SF68. The amount of probiotics and prebiotics to be administered to the animal is determined by the skilled artisan based upon the type and nature of the prebiotic and probiotic and the type and nature of the animal, e.g., the age, weight, general health, sex, extent of microbial depletion, presence of harmful bacteria, and diet of the animal. Generally, probiotics are administered to the animal in amounts of from about one to about twenty billion colony forming units (CFUs) per day for the healthy maintenance of intestinal microflora, preferably from about 5 billion to about 10 billion live bacteria per day. Generally, prebiotics are administered in amounts sufficient to positively stimulate the healthy microflora in the gut and cause these "good" bacteria to reproduce. Typical amounts are from about one to about 10 grams per serving or from about 5% to about 40% of the recommended daily dietary fiber for an animal. The probiotics and prebiotics can be made part of the composition by any suitable means. Generally, the agents are mixed with the composition or applied to the surface of the composition, e.g., by sprinkling or spraying. When the agents are part of a kit, the agents can be admixed with other materials or in their own package.

The compositions may further comprise additional ingredients such as vitamins, minerals, fillers, palatability enhancers, binding agents, flavors, stabilizers, emulsifiers, sweeteners, colorants, preservatives, buffers, salts, coatings, amino acids, enzymes, coenzymes, and the like known to skilled artisans. Minerals useful in such compositions include calcium, phosphorous, potassium, sodium, iron, chloride, boron, copper, magnesium, manganese, iodine, selenium, and the like. Vitamins useful in such compositions include such fat soluble vitamins as A, B, C, D, E, and K. Stabilizers include substances that tend to increase the shelf life of the composition such as preservatives, synergists and sequestrants, packaging gases, stabilizers, emulsifiers, thickeners, gelling agents, and humectants. Examples of emulsifiers and/or thickening agents include gelatin, cellulose ethers, starch, starch esters, starch ethers, and modified starches. Specific amounts of such ingredients will depend on a variety of factors such as the particular ingredients, intended use, and the like.

In another embodiment, the compositions further comprise ingredients needed to produce dietary supplements. The dietary supplements comprise a high concentration of one or more of melatonin, carotenoids, and zinc. This permits the supplement to be administered to the animal in small amounts, or in the alternative, permits the supplement to be diluted before administration to an animal. The dietary supplement may require admixing with water or other diluent prior to administration to the animal.

In some embodiments, the compositions further comprise one or more comestible ingredients useful for producing comestible compositions, e.g., food compositions and beverage compositions. In certain embodiments, the food compositions comprise a macronutrient composition suitable for the type of food being designed. In one embodiment, a food composition has about 20 to 32% protein, about 30 to 50% carbohydrate, about 5% to 20% fat, and about 5% to 25% moisture. In another embodiment, the food composition is a pet food composition such as a premium or super-premium pet food composition. In one embodiment, the pet food is formulated for canines and has a protein content of about 20 to 30%, preferably about 24 to 29%, and more preferably about 25 to 28%. In another embodiment, the food composition is formulated for felines and has a protein content of about 35 to 45%, preferably about 37 to 42%, and more preferably about 39 to 41%. In a preferred embodiment, the composition is a food composition comprising melatonin, carotenoids, and zinc, and further comprising about 15% to about 50% protein, about 5% to about 40% fat, about 5% to about 10% ash content, and having a moisture content of about 5% to about 20%. The protein can be derived from vegetable proteins such as soybean meal, soy protein concentrate, corn gluten meal, wheat gluten, cottonseed, and peanut meal, or animal proteins such as casein, albumin, and meat protein. Examples of meat protein useful herein include pork, lamb, equine, poultry, fish, beef, and mixtures thereof. Examples of carbohydrates include grains or cereals such as rice, corn, milo, sorghum, alfalfa, barley, soybeans, canola, oats, wheat, and mixtures thereof. Fats are typically animal fats such as tallow. The food compositions may also ly comprise other materials such as dried whey and other dairy by-products. In some embodiments, the ash content of such food compositions ranges from less than 1% to about 15%, preferably from about 5% to about 10%.

In a preferred embodiment, the food composition is a complete and nutritionally balanced pet food. In this embodiment, the pet food may be a "wet food", "dry food", or "intermediate moisture food." "Wet food" describes pet food that is typically sold in cans or foil bags, and has a moisture content typically in the range of about 70% to about 90%. "Dry food" describes pet food that is of a similar composition to wet food, but contains a limited moisture content, typically in the range of about 5% to about 15% or 20%. Dry food is usually in the form of biscuits or kibbles. Dry food products include a variety of foods of various moisture contents, such that they are relatively shelf-stable and resistant to microbial or fungal deterioration or contamination. Also preferred are dry food compositions that are extruded food products such as pet foods or snack foods for either humans or companion animals. "Intermediate moisture food" describes pet food that has a moisture content typically in the range of about 20% to about 50%.

The food compositions may also comprise one or more fiber sources. The term "fiber" includes all sources of "bulk" in the food whether digestible or indigestible, soluble or insoluble, fermentable or nonfermentable. Preferred fibers are from plant sources such as marine plants but microbial sources of fiber may also be used. A variety of soluble or insoluble fibers may be utilized, as will be known to those of ordinary skill in the art. The fiber source can be beet pulp (from sugar beet), gum arabic, gum talha, psyllium, rice bran, carob bean gum, citrus pulp, fructooligosaccharide, pectin, short chain oligofructose, mannanoligofructose, soy fiber, arabinogalactan, galactooligosaccharide, arabinoxylan, or mixtures thereof. Alternatively, the fiber source can be a fermentable fiber. Fermentable fiber has previously been described to provide a benefit to the immune system of a companion animal.

As defined herein, the food compositions are formulated for a companion animal, *e.g.,* a dog or cat.

In various embodiments, the melatonin, carotenoids, and zinc are pre-blended with other ingredients used to manufacture a food composition, e.g., a blend of vitamins and minerals or palatability enhancers. In other embodiments, the melatonin, carotenoids, and zinc are used to coat a food composition. In one embodiment, the melatonin, carotenoids, and zinc are added to a food or beverage composition just prior to offering it to the animal, e.g., by sprinkling the composition onto the food composition or admixing the composition with water.

In certain embodiments, compositions comprising melatonin, carotenoids, and zinc are preferably used with a high-quality commercial food. As used herein, "high-quality commercial food" refers to a food manufactured to produce the digestibility of the key nutrients of 80% or more, as set forth in, *e.g.,* the recommendations of the National Research Council for dogs, or in the guidelines set by the Association of American Feed Control Officials. Similar high nutrient standards would be used for other animals.

The food compositions and dietary supplements may be specially formulated for the intended recipients or consumers, such as for adult animals or for older or young animals. For example, a food composition adapted for puppies or kittens or adapted for active, pregnant, lactating, or aging animals can be prepared. In general, specialized compositions will comprise energy and nutritional requirements appropriate for animals at different stages of development or age.

The skilled artisan will understand how to determine the appropriate amount of melatonin, carotenoids, zinc, and any other ingredients to be used to produce a given comestible composition, dietary supplement, or other similar composition. The skilled formulator may consider important the animal's species, age, size, weight, health, and the like in determining how best to formulate a particular food composition, food supplement, or pharmaceutical composition comprising the melatonin, carotenoids, zinc, and other components. Other factors that may be taken into account for formulation include the type of composition (e.g., pet food composition versus dietary supplement), the desired dosage of each component (melatonin, carotenoids, and zinc), the average consumption of specific types of compositions by different animals (e.g., based on species, body weight, activity/energy demands, and the like) and the manufacturing conditions under which the composition is prepared. Preferably, the concentrations of melatonin, carotenoids, and zinc to be added to the composition are calculated on the basis of the nutrient requirements of the animal.

In an alternative embodiment, the amount of melatonin, carotenoids, and zinc in the composition is a function of an amount required to establish specified concentrations or a desired range of concentrations of melatonin, carotenoids, and zinc, in the blood of the animal. The specified concentrations, or desired ranges of melatonin, carotenoids, and zinc in the blood may be calculated by determining the blood levels of animals fed the recommended amounts of melatonin, carotenoids, and zinc specified above, as would be appreciated by a skilled artisan.

In another aspect, pharmaceutical compositions comprising a composition of the present invention and one or more pharmaceutically-acceptable carriers, diluents, or are also described. Generally, pharmaceutical compositions are prepared by admixing a compound or composition with excipients, buffers, binders, plasticizers, colorants, diluents, compressing agents, lubricants, flavorants, moistening agents, and the like, including other ingredients known to skilled artisans to be useful for producing pharmaceuticals and formulating compositions that are suitable for administration to an animal as pharmaceuticals.

In another aspect, nutraceutical compositions comprising a composition of the present invention and one or more nutraceuticals. are also described.

In another aspect, methods for enhancing cognitive function in an animal. are also described. The methods comprise administering a composition comprising melatonin and one or more carotenoids to the animal in an amount effective for enhancing cognitive function in the animal. In certain embodiments, the compositions further comprise zinc.

In another aspect, methods for enhancing cognitive function in an animal. are also described. The methods comprise administering to an animal a composition comprising one or more carotenoids and one or more compounds that can be metabolized to produce melatonin to the animal in an amount effective for enhancing cognitive function in the animal. The supplements are converted to melatonin by the metabolic processes in the animals and the melatonin is involved in enhancing cognitive function as described herein. In preferred embodiments, the supplements are serotonin, tryptophan, and 5-hydorxytryptophan. The supplements can be administered in any amount sufficient to obtain the melatonin amounts required herein upon metabolism. Typically, the supplements are administered in a composition comprising one or more carotenoids and supplements. In certain embodiments, the compositions further comprise zinc in amounts effective for enhancing cognitive function.

The melatonin, carotenoids, and zinc can be administered to the animal together or in conjunction in various combinations. Administration can be on an as-needed or as-desired basis of varying or regular frequency. A goal of regular administration is to provide the animal with a regular and consistent dose of the composition or the direct or indirect metabolites that result from such administration. Such regular and consistent dosing will tend to create constant blood levels of the components of the compositions or their direct or indirect metabolites. Thus, regular administration can be once monthly, once weekly, once daily, or more than once daily. Similarly, administration can be every other day, week, or month, every third day, week, or month, every fourth day, week, or month, and the like. Administration can be multiple times per day. When utilized as a supplement to ordinary dietetic requirements, the composition may be administered directly to the animal, e.g., orally. The compositions can alternatively be contacted with, or admixed with, daily food, including a fluid such as drinking water. Administration can also be carried out as part of a dietary regimen for the animal. For example, a dietary regimen may comprise causing the regular ingestion by the animal of any composition described herein in an amount effective for enhancing cognitive function. Preferably, the compositions are administered to the animal in the evening, *e.g.,* between 3PM and 10PM, and/or can be available throughout the evening and overnight.

Administration of the compositions, including administration as part of a dietary regimen, can span a period of time ranging from parturition through the adult life of the animal. In certain embodiments, the animal is a young or growing animal. In preferred embodiments, the animal is an aging animal.

A composition or method for enhancing cognitive function has many effects on an animal. Many day-to-day life functions are dependent upon or related to cognitive function. For example, enhancing cognitive function is related to preventing a decline of social interaction, reducing or preventing age-related behavioral changes, increasing trainability, improving attention, maintaining optimal brain function, facilitating learning and memory, reducing memory loss, and preventing or treating cognitive decline caused by dementia in an animal. Similarly, enhancing cognitive function promotes the overall health and wellness of an animal. Additionally, enhancing cognitive function improves the quality of life for the animal. Further, enhancing cognitive function is integral to extending the prime for an animal.

In one embodiment, the composition is administered to the animal in conjunction with one or more cognitive drugs in an amount effective for enhancing cognitive function as defined herein. In a particular embodiment, the composition administered is the pharmaceutical composition that includes a cognitive drug along with melatonin, one or more carotenoids, and zinc. In a preferred embodiment, the composition is administered to the animal on a daily basis, preferably in a single dose.

In certain embodiments, the animal is a healthy aging animal. In others, the animal has a phenotype associated with age-related cognitive impairment. For example, when compared to a control animal not having the phenotype, the animal may have a phenotype that includes one or more of decreased ability to recall, short-term memory loss, decreased learning rate, decreased capacity for learning, decreased problem solving skills, decreased attention span, increased confusion, or dementia (Alzheimer's in humans or its equivalent in other animals).

The disclosure shows methods for affecting and enhancing various cognitive related functions. Generally, the methods comprise administering the compositions of the present invention to an animal in amounts defined herein for the compositions and methods.

The disclosure also shows methods for reducing or preventing a decline of social interaction in an animal comprising administering a composition comprising melatonin and one or more carotenoids to the animal in an amount effective to reduce or prevent a decline in social interaction. In preferred embodiments, the compositions further comprise zinc in amounts effective for reducing or preventing a decline of social interaction in an animal. The methods ensure that an aging animal remains involved in playtime, participates in group activities, interacts with caregivers, and the like.

In another aspect, the disclosure shows methods for reducing or preventing age-related behavioral changes in an animal comprising administering melatonin and one or more carotenoids to the animal in an amount effective for reducing or preventing age-related behavioral changes in the animal. Preferably, the age-related behavioral changes are one or more of forgetfulness, disorientation, reduced social interaction, changes in sleep and wake habits (particularly an increase in nighttime activity), loss of "housetraining" that results in changes in urination and defecation locations and patterns, confusion, frustration, increasing daytime activity, change in temperament such as agitation and aggression, pacing, and wandering. Such changes have been noted in response to cognitive declines in humans and other animals. The compositions further comprise zinc in amounts effective for reducing or preventing age-related behavioral changes in an animal.

In another aspect, the disclosure shows methods for increasing trainability of an animal comprising administering a composition comprising melatonin and one or more carotenoids to the animal in an amount effective for increasing trainability. For example, administering the composition while "potty training" babies, puppies, and kittens permits the animal to learn the task more quickly than if the training occurred without using the composition. Similarly, training a dog or cat to obey verbal, signal, or other commands permits the animal to learn the task more quickly than if the training occurred without using the composition. Similarly, the compositions are useful for training feral or wild animals such as animals used in a circus or pets raised in the wild. In preferred embodiments, the compositions further comprise zinc in amounts effective for increasing trainability of an animal.

In another aspect, the disclosure also depicts methods for maintaining optimal brain function in an animal. The methods comprise administering a composition comprising melatonin and one or more carotenoids to the animal in an amount effective to prevent or delay a decline in brain function, particularly over time. In preferred embodiments, the compositions further comprise zinc in amounts effective for maintaining optimal brain function in an animal. Generally, the methods ensure that an aging animal maintains healthy and optimal brain function throughout life and has a better quality of life, particularly in older animals. The methods also slow the progression of mental decline in aging animals such as dogs.

In a further aspect, the diclosure shows methods for facilitating learning and memory in an animal. The methods comprise administering a composition comprising melatonin and one or more carotenoids to the animal in an amount effective for facilitating learning and memory in the animal. In one embodiment, the animal is an aging animal. In another, the animal has reached from about 60% to about 90% of its life expectancy, preferably about 75% of its life expectancy. The methods help aging animals remember facts and understand instructions. In preferred embodiments, the compositions further comprise zinc in amounts effective for facilitating learning and memory in an animal.

In another aspect, the diclosure showsmethods for reducing memory loss in an animal. The methods comprise administering a composition comprising melatonin and one or more carotenoids to the animal in an amount effective to reduce memory loss over time. In preferred embodiments, the compositions further comprise zinc in amounts effective for reducing memory loss in an animal.

In another aspect, the diclosure showsmethods for preventing or treating cognitive decline caused by dementia in an animal. The methods comprise administering a composition comprising melatonin and one or more carotenoids to the animal in an amount effective to prevent or treat cognitive decline caused by dementia. In preferred embodiments, the compositions further comprise zinc in amounts effective for preventing or treating cognitive decline caused by dementia in an animal. Dementia can be Alzheimer's disease (AD) in humans, Canine Cognitive Dysfunction Syndrome (CCDS) in canines, or similar diseases in other animals. The methods are based upon the discovery that the compositions and methods of the present invention prevent or reduce cognitive decline caused by dementia by reducing the effects on cognition of damage that results from the causes of dementia, e.g., amyloid deposits or deterioration of artery function.

In another aspect, the diclosure showsmethods for maintaining mental clarity and alertness in an animal. The methods comprise administering a composition comprising melatonin and one or more carotenoids to the animal in an amount effective for maintaining mental clarity and alertness. In preferred embodiments, the compositions further comprise zinc in amounts effective for maintaining mental clarity and alertness in an animal.

In one aspect, the diclosure showsmethods for promoting the health and wellness of an animal comprising administering a composition comprising melatonin and one or more carotenoids to the animal in an amount effective to promote health and wellness. In preferred embodiments, the compositions further comprise zinc in amounts effective for promoting health and wellness.

In one aspect, the diclosure showsmethods for improving the quality of life for an animal comprising administering a composition comprising melatonin and one or more carotenoids to the animal in an amount effective to improve the quality of life for the animal. In preferred embodiments, the compositions further comprise zinc in amounts effective for improving the quality of life.

In a further aspect, the diclosure showsmethods for extending the prime for an animal comprising administering a composition comprising melatonin and one or more carotenoids to the animal in an amount effective for extending the prime for the animal. In preferred embodiments, the compositions further comprise zinc in amounts effective for extending the prime for the animal.

In the methods for reducing or preventing a decline of social interaction, reducing or preventing age-related behavioral changes, increasing trainability, improving attention, maintaining optimal brain function, facilitating learning and memory, reducing memory loss, preventing or treating cognitive decline caused by dementia, maintaining mental clarity and alertness, promoting health and wellness, improving quality of life, and extending the prime, the animal is preferably a human or companion animal, most preferably a dog or cat. The amounts of melatonin, carotenoids, zinc, and other ingredients used in these methods are the same as the amounts or within the ranges given herein for enhancing cognitive function.

In a further aspect, the diclosure showskits suitable for administering a composition comprising melatonin and one or more carotenoids to an animal. The kits comprise in separate containers in a single package or in separate containers in a virtual package, as appropriate for the kit component, (a) one or more melatonins from natural or synthetic sources and (b) one or more carotenoids and one or more of (1) one or more comestible ingredients; (2) zinc, (3) one or more cognitive drugs; (4) one or more prebiotics; (5) one or more probiotics; (6) one or more diagnostic devices suitable for determining whether an animal could benefit from compositions and methods for enhancing cognitive function; (7) instructions for how to combine or prepare the melatonins and carotenoids and any other ingredients provided in the kit for administration to an animal; (8) instructions for how to use the combined kit components, prepared kit components, or other kit components for the benefit of an animal; and (9) a device for administering the combined or prepared kit components to an animal. The components are each provided in separate containers in a single package or in mixtures of various components in different packages. In preferred embodiments, the kits comprise the melatonin, astaxanthin, and zinc. The kits may comprise the ingredients in various combinations. For example, the kit could comprise a mixture of one or more melatonin sources and one or more carotenoids in one container and one or more other ingredients, e.g., a zinc supplement, in one or more other containers. Similarly, the kit could comprise a mixture of synthetic melatonin and zinc in one container and one or more other ingredients in one or more other containers. Other such combinations can be produced by the skilled artisan based upon the characteristics of the ingredients and their physical and chemical properties and compatibilities.

In another aspect, the diclosure showsa means for communicating information about or instructions for one or more of (1) using compositions of the present invention for enhancing cognitive function; (2) admixing the melatonin, carotenoids, zinc, and other components to produce a composition suitable for enhancing cognitive function; (3) using the kits of the present invention for enhancing cognitive function; (4) benefits of using the compositions for enhancing cognitive function; and (5) administering the compositions to an animal. The means comprises one or more of a physical or electronic document, digital storage media, optical storage media, audio presentation, audiovisual display, or visual display containing the information or instructions. Preferably, the means is selected from the group consisting of a displayed website, a visual display kiosk, a brochure, a product label, a package insert, an advertisement, a handout, a public announcement, an audiotape, a videotape, a DVD, a CD-ROM, a computer readable chip, a computer readable card, a computer readable disk, a USB device, a FireWire device, a computer memory, and any combination thereof.

In another aspect, the diclosure showsmethods for manufacturing a food composition comprising melatonin, carotenoids, zinc, and one or more other ingredients suitable for consumption by an animal, e.g., protein, fat, carbohydrate, fiber, minerals, and vitamins. The methods comprise admixing one or more ingredients suitable for consumption by an animal with melatonin, carotenoids, zinc, and possibly other ingredients. Alternatively, the methods comprise applying melatonin, carotenoids, zinc, and other ingredients if desired, separately or in any combination onto the food composition, e.g., as a coating or topping. The melatonin, carotenoids, and zinc can be added at any time during the manufacture and/or processing of the food composition. This includes, for example, admixing the melatonin, carotenoids, and zinc as part of the core formulation of the "body" of the food composition or applying them as a coating, *i.e.,* primarily to the surface of the food composition after its manufacture. The compositions can be made according to any method suitable in the art.

In another aspect, the present diclosure showsa package comprising a composition of the present invention and a label affixed to the package containing a word or words, picture, design, acronym, slogan, phrase, or other device, or combination thereof, that indicates that the contents of the package contains a composition suitable for enhancing cognitive and related functions such as reducing or preventing a decline of social interaction, reducing or preventing age-related behavioral changes, increasing trainability, improving attention, maintaining optimal brain function, facilitating learning and memory, reducing memory loss, preventing or treating cognitive decline caused by dementia, maintaining mental clarity and alertness, promoting health and wellness, improving quality of life, and extending the prime, particularly an aging animal. Typically, such device comprises the words "enhances cognitive function", "improves memory", "reduces memory loss in aging animals", "maintains mental clarity and alertness" or an equivalent expression printed on the package. Any package or packaging material suitable for containing the composition is useful in the invention, e.g., a bag, box, bottle, can, pouch, and the like manufactured from paper, plastic, foil, metal, and the like. In a preferred embodiment, the package contains a food composition adapted for a particular animal such as a human, canine or feline, as appropriate for the label, preferably a companion animal food composition.

In another aspect, the diclosure showsfor use of melatonin and one or more carotenoids, and zinc, to prepare a medicament for enhancing cognitive and related functions such as reducing or preventing a decline of social interaction, reducing or preventing age-related behavioral changes, increasing trainability, improving attention, maintaining optimal brain function, facilitating learning and memory, reducing memory loss, and maintaining mental clarity and alertness in an animal. Generally, medicaments are prepared by admixing a compound or composition with excipients, buffers, binders, plasticizers, colorants, diluents, compressing agents, lubricants, flavorants, moistening agents, and other ingredients known to skilled artisans to be useful for producing medicaments and formulating medicaments that are suitable for administration to an animal.

The compositions described herein, including the comestible compositions, nutraceutical compositions, pharmaceutical compositions, and medicaments, are administered to the animal using a variety of administration routes. Such routes include oral, intranasal, intravenous, intramuscular, intragastric, transpyloric, subcutaneous, rectal, and the like if appropriate for the particular composition. Preferably, the compositions are administered orally.

The compositions described herein, including the comestible compositions, nutraceutical compositions, pharmaceutical compositions, and medicaments are administered to the animal for a time required to accomplish one or more objectives of the invention, e.g., enhancing cognitive function, reducing or preventing a decline of social interaction, reducing or preventing age-related behavioral changes, increasing trainability, improving attention, maintaining optimal brain function, facilitating learning and memory, reducing memory loss, preventing or treating cognitive decline caused by dementia, maintaining mental clarity and alertness, promoting health and wellness, improving quality of life, and extending the prime in an animal. The compositions are suitable for long-term administration or administration on any schedule compatible with the composition and objective.

### EXAMPLES

The invention can be further illustrated by the following examples, although it will be understood that the examples are included merely for purposes of illustration and are not intended to limit the scope of the invention unless otherwise specifically indicated.

### Example 1

### Animal and Test Groups

Sixty (60) dogs at least 9 years of age were selected based on memory performance on a variable delay Delayed Non-Matching to Position (DNMP) analysis. Subsequently, the dogs were placed in 3 non-overlapping groups defined as Low Performing Memory (LPM), N=24; Moderate Performing Memory (MPM), N=24; and High Performing Memory (HPM), N=12. The LPM and MPM groups were further broken down into treatment and control groups to give a total of five treatment groups:
MPM - Control Composition AM/PM (N=12)
MPM - Control Composition AM/Text Composition PM (N=12)
LPM - Control Composition AM/PM (N=12)
LPM - Control Composition AM/Test Composition PM (N=12)
HPM - Control Composition AM/PM (N=12)

The animals were fed a Control Composition (CC) and a Test Composition (TC). The CC was a complete and balanced dog food. The TC was the CC containing 13.5 mg per kg (mg/kg) melatonin, 8.2 mg/kg astaxanthin, and 208 mg/kg zinc. The compositions had a nutrient concentration of 28.0% Crude Protein, 12.0% Crude Fat, and 3.0% Crude Fiber. The animals were fed the CC and the TC twice daily for 77 days. The feedings occurred at 9 AM (± 30 minutes) and 6 PM (± 30 minutes). The animals fed the TC were fed the CC at 9 AM and the TC at 6 PM.

After a 6 day wash-in, animals were tested on 3 cognitive assessment tasks: the attention task (over 30 days), varDNMP (over 14 days), and motor learning task (over 10 days).

Animals were tested using an "attention task" procedure during days 7 to 36. The attention task procedure was adapted from an oddity discrimination learning task in which dogs are trained to select the odd object out of three. The purpose of this modification was to further challenge canine attention. This task required the adoption of a specially modified test tray that contains four food wells. The preferred object of each animal identified through the preference test was the rewarded object for the acquisition phase of testing.

For the acquisition phase, the animals were initially trained on a two-choice object discrimination problem that required them to learn that choosing a certain object was always associated with a food reward. The acquisition criterion was divided into 2 stages. The first stage required animals to perform above 90% accuracy one day or above 80% accuracy over two consecutive test days. The second stage required animals to perform above 70% accuracy over three consecutive days subsequent to passing the first stage criterion. Each test session consisted of 10 trials. A maximum of 20 sessions was given (1 preference test and 19 acquisition sessions).

After animals passed the acquisition phase, they were tested on the test phase of the attention task. In the test phase, the object that was rewarded during the acquisition phase was always rewarded. Animals were tested with 1, 2, 3, or 4 objects. Each test session consisted of 12 trials. The number of objects was varied pseudo-randomly and occurred equally over the 12 trials. For the first 5 sessions (1 to 5), the negative stimulus used in the acquisition phase served as the "same" negative stimulus on the attention task (referred to as "same" task). For sessions 6 to 10, the positive stimulus remained unchanged. However, the negative stimulus was switched to a "different" object that was more similar to the rewarded object (referred to as the "different" task).

### Cognitive Assessment

### Acquisition of Two-Choice Discrimination as a Function of Cognitive Grouping

To establish whether the baseline groupings generalized to performance on the two-choice discrimination, the control animals from each of the 3 groups were compared using a one-way ANOVA. The results are shown in Table 1.

**Table 1**

| DNMP Groups | % Correct |
|---|---|
| HMP | 70.5 |
| MMP | 74.1 |
| LMP | 67.5 |

Referring to Table 1, the results show that the LPM animals performed more poorly than the other two groups (P=0.428). Moreover, the animals classified as cognitively MPM performed better overall than the animals classified as HPM.

### Effects of TC on Acquisition of Two-Choice Discrimination Tasks

### Comparison of Control and Treatment Groups.

In the initial analysis, a t-test was used to compare accuracy in acquisition of animals fed the CC with that of animals fed the TC, independently of group classification. The results are shown in Table 2.

**Table 2**

| Food Groups | % Correct |
|---|---|
| CC | 70.7 |
| TC | 76.6 |

Referring to Table 2, the results of the analysis showed a significant effect (P=0.097) and a significant difference (P=0.0485) using a one-tailed test. The data shows that that the animals fed the CC made more errors in learning the task than did the animals fed the TC.

### Effects of Cognitive Classification and Treatment

The effect of prior grouping and food was examined using a factorial ANOVA with treatment and cognitive classification serving as between-subject variables. The results are shown in Table 3.

**Table 3**

| | % Correct | |
|---|---|---|
| DNMP Groups | CC | TC |
| MMP | 74.1 | 78.3 |
| LMP | 67.5 | 74.9 |

Referring to Table 3, the analysis showed a significant main effect of cognitive classification (p=0.039). The treatment effect did not achieve statistical significance (p=0.1318). The data shows poorer performance by the LPM group than the MPM group and poorer performance by the animals fed the CC when compared with the animals fed the TC. This means that the animals fed the TC learned more accurately than the animals fed the CC.

To understand further the factors affecting learning of the 2-choice discrimination task, an additional analysis was performed taking age and treatment into consideration. The groups were separated into three age groups; 8 to 9.99 (N=19), 10 to 11.99 (N=18), and 12 and greater (N=22). A factorial ANOVA was performed with errors to criterion as the dependent variable and both age group and treatment condition as factors. The results are shown in Table 4.

**Table 4**

| | % Correct | |
|---|---|---|
| Age Groups | CC | TC |
| 8-10 yrs | 74.6 | 81.4 |
| 10-12 yrs | 74.5 | 78.5 |
| > 12 yrs | 64.6 | 72.5 |

Referring to Table 4, the data shows a significant effect of age (p=0.0185) and a significant effect of treatment (p=0.082). Poorer performance occurred with increasing age. Within age groups, the animals on the TC learned with fewer errors than the animals on the CC.

### Attention Task

Effect of Distracters, Diet, and Cognitive Grouping.

The initial analysis compared performance as a function of number of distracters of the LPM and MPM groups on the TC and the CC. Because in almost all instances the animals performed without errors when presented with a single distracter, the analysis only considered the cases with 1, 2, or 3 distracters.

An omnibus repeated measures ANOVA was performed in which task (same vs. different) and number of distracters (1 to 3) were within-subject variables, and baseline grouping (LPM, MPM, or successful ager) and treatment were between-subject variables.

The results are shown in Table 5 for the combination of data from both the "same" and "different" object testing.

**Table 5**

| | % Correct | | |
|---|---|---|---|
| | 1 Distractor | 2 Distractors | 3 Distractors |
| CC | 73.3 | 54.4 | 50.6 |
| TC | 77.1 | 64.6 | 60.0 |

Referring to Table 5, the analysis showed significant main effects of treatment (p=0.032), task (p=0.000) and distracter (p=0.00). There was also a significant interaction between task and distracter (p=0.0478) and a marginally significant between number of distracters and treatment (p=0.083). The results show that animals fed the TC made fewer errors than the animals fed the CC and that performance of both groups became worse with increases in the of number of distracters. The data also shows that composition effect was largely driven by differences in accuracy when there were 2 or three distracters and that this accounts for the marginally significant interaction between number of distracters and treatment.

### Comparison of Three Groups as a Function of Baseline Cognitive Characterization

The next analysis looked at the effect of cognitive classification and was based only on the performance of the animals fed the CC. The data were analyzed with a repeated-measures ANOVA with cognitive classification (MPM, LPM and HPM) as a between-subject variable and distracter as a within-subject variable. The results are shown in Table 6.

**Table 6**

| | % Correct | | |
|---|---|---|---|
| | 1 Distractor | 2 Distractors | 3 Distractors |
| HMP | 69.26 | 52.7 | 44.22 |
| MMP | 68.81 | 42.78 | 45.98 |
| LMP | 71.74 | 43.93 | 40.09 |

Referring to Table 6, there were significant effects of distracters, but no effect of classification.

### Effect of Age on Performance of Attention Task

To examine the contribution of age to performance on the attention task, separate analyses of the "same" and "different" tasks was performed, using repeated measures ANOVAs with number of distracters as a within-subject variable, and both age group and food as between-subject variables. The results are shown in Table 7.

**Table 7**

| | | % Correct | | |
|---|---|---|---|---|
| Age Groups | | 1 Distractor | 2 Distractors | 3 Distractors |
| 8-10 yrs | CC | 71.7 | 58.1 | 46.8 |
| | TC | 69.3 | 44.0 | 45.3 |
| 10-12 yrs | CC | 73.9 | 39.8 | 38.9 |
| | TC | 73.7 | 61.5 | 56.5 |
| > 12 yrs | CC | 64.5 | 42.7 | 43.7 |
| | TC | 67.0 | 58.1 | 49.5 |

Referring to Table 7, the results from the "same" condition showed significant main effects of distracters (p=0.00) and treatment (p=0.0343). The age effect had a significance of p=0.0510. The same analysis for the "different" task only showed a significant effect of distracters. In this analysis, the interaction between distracters and treatment was significant (p=0.094). The results are shown in Table 8.

**Table 8**

| | | % Correct | | |
|---|---|---|---|---|
| Age Groups | | 1 Distractor | 2 Distractors | 3 Distractors |
| 8-10 yrs | CC | 80.7 | 73.9 | 61.8 |
| | TC | 84.0 | 81.3 | 69.7 |
| 10-12 yrs | CC | 78.9 | 58.5 | 56.0 |
| | TC | 88.5 | 72.3 | 69.4 |
| > 12 yrs | CC | 71.0 | 56.7 | 56.2 |
| | TC | 77.7 | 65.4 | 63.5 |

### Correlations between Age and Attention Data with "Same" Object Task

The origins of the age effect for the same task show that errors increased as a function of age and that the correlation was higher when there were 1 and 2 distracters than 3. With 1 distracter, the correlation was r=0.32, P<0.05; with 2 distracters, the correlation was 0.4, P<0.05. The correlation for 3 distracters was r=0.22.

### Correlations between Age and Attention Data with "Different" Object Task

The results from the difference task show a smaller positive correlation with age, with errors increasing with increasing age.

### Effect of Treatment, Number of Distracters, and Cognitive Classification on

### Response Latency in the Attention Task

Most of the dogs responded rapidly on all trials. In analyzing response latencies, a few animals showed average response latencies that were considerably higher than the remaining animals. If the data from these animals were included, it would have markedly distorted the grouped data. Accordingly, all animals that had mean latencies that were greater than 2 SD units from the combined group mean were removed from the analysis. One animal from each group was dropped; the animals removed included Sealia (LPM; TC), Nettie (LPM; CC), Chilli (MPM; TC), Cherry HMP; CC). The data were first analyzed with a repeated measures ANOVA with task and distracters (o to 3) as within-subject variables and cognitive classification and treatment as between-subject variables. The results are shown in Table 9.

**Table 9**

| | Mean Latency in Seconds | | | |
|---|---|---|---|---|
| | 0 Distractors | 1 Distractor | 2 Distractors | 3 Distractors |
| Same task | 1.74 | 1.84 | 1.83 | 2.00 |
| Different task | 1.69 | 1.70 | 1.84 | 2.11 |

Referring to Table 9, the data show a significant main effect of distracter (p=o.ooo), and no other significant effects or interactions. The significant effect of distracters reflected a general increase in latency with increasing number of distracters. The results were similar for both tasks.

### Effect of Treatment on Response Latency in the Attention Task

Because of an absence of any effect of cognitive classification, a separate analysis comparing the treatment groups, independent of cognitive classification, was performed. The data were analyzed with a repeated measures ANOVA with both task and number of distracters as within-subject variables and diet as the between-subject variable. The results showed a significant effect of number of distracters (p=o.ooo), and marginally significant effects of diet (p=0.0879), diet by number of distracters (p=0.088) and task by number of distracters (p=0.059). The results are shown in Table 10. The results show that (1) the animals fed the CC responded more slowly on average than the animals fed the TC, (2) the differences between the groups are greater with increasing number of distracters, and (3) the differences between the groups are greater in the same task than in the different task.

**Table 10**

| | | Mean Latency in Seconds | | | |
|---|---|---|---|---|---|
| | | 0 Distractors | 1 Distractor | 2 Distractors | 3 Distractors |
| Same Task | CC | 2.00 | 2.20 | 2.19 | 2.50 |
| | TC | 1.76 | 1.80 | 1.74 | 1.90 |
| Different Task | CC | 1.85 | 1.98 | 2.20 | 2.35 |
| | TC | 1.66 | 1.60 | 1.76 | 1.90 |

Given all the results, the data shows that the TC improved cognitive performance as assessed by improved learning during a new object discrimination task, improved latency in object discrimination, and improved attention performance.

In the specification, there have been disclosed typical preferred embodiments of the invention. The scope of the invention is set forth in the claims.

## Claims

1. A composition suitable for enhancing cognitive function in a companion animal comprising melatonin and one or more carotenoids and zinc in an amount effective for enhancing cognitive function in the companion animal, wherein the composition comprises from 0.1 to 80 mg/kg melatonin, from 0.01 to 20 mg/kg of one or more carotenoids.

2. The composition of claim 1 comprising from about 0.1 to 400 mg/kg zinc.

3. A composition comprising melatonin, one or more carotenoids and zinc in an amount effective for enhancing cognitive function for use in preventing, reducing, or delaying a decline in cognitive functions in a companion animal, wherein the composition is to be administered to the companion animal in an amount effective for enhancing cognitive function in the companion animal, wherein the composition comprises from 0.1 to 80 mg/kg melatonin, from 0.01 to 20 mg/kg of one or more carotenoids and from 0.1 to 400 mg/kg zinc, and wherein the companion animal is selected from a canine or a feline.

4. The composition for use according to claim 3 wherein the melatonin is a synthetic melatonin.

5. The composition for use according to claim 3 wherein the carotenoids are xanthophylls; optionally wherein the xanthophylls are selected from the group consisting of astaxanthin, lutein, zeaxanthin, neoxanthin, violaxanthin, a-cryptoxanthin, and β-cryptoxanthin; optionally wherein the xanthophyll is astaxanthin.

6. The composition for use according to claim 3 wherein the canine or feline is an aging canine or feline.

7. The composition for use according to claim 3 wherein the canine or feline is an canine or feline susceptible to or suffering from a decline in cognitive function brought about by the aging process or by injury or disease.

8. The composition for use according to claim 3 wherein the composition is administered to the canine or feline in conjunction with one or more cognitive drugs in an amount effective for enhancing cognitive function; optionally wherein the cognitive drug is one or more of selegiline, nicerogoline, a phosphatidylserine, propentofyline, galantamine, vinpocetine, donepezil, a *Ginkgo biloba* extract, a bisphosophonate, raloxifene, an estrogen, a phytoestrogen, calcitonin, risedronate, or alendronate.

9. The composition for use according to claim 3 wherein the canine or feline is an aging canine or feline ; optionally wherein the canine or feline has a phenotype associated with age-related cognitive impairment; optionally wherein the phenotype includes one or more of decreased ability to recall, short-term memory loss, decreased learning rate, decreased capacity for learning, decreased problem solving skills, decreased attention span, increased confusion, or dementia, as compared to a control canine or feline not having the phenotype.

10. A composition comprising melatonin, one or more carotenoids and zinc in an amount effective for reducing or preventing age-related behavioral changes for use in preventing, reducing, or delaying a decline in cognitive functions in a canine or feline , wherein the composition is administered to the canine or feline in an amount effective for reducing or preventing age-related behavioral changes in the canine or feline , wherein the composition comprises from 0.1 to 80 mg/kg melatonin, from 0.01 to 20 mg/kg of one or more carotenoids and from 0.1 to 400 mg/kg zinc,

## Patentansprüche

1. Zusammensetzung, die zum Verbessern der kognitiven Funktion in einem Haustier geeignet ist, umfassend Melatonin und ein oder mehrere Carotenoide und Zink, in einer zum Verbessern der kognitiven Funktion in einem Haustier wirksamen Menge, wobei die Zusammensetzung von 0,1 bis 80 mg/kg Melatonin, von 0,01 bis 20 mg/kg von einem oder mehreren Carotenoiden umfasst.

2. Zusammensetzung nach Anspruch 1, umfassend von etwa 0,1 bis 400 mg/kg Zink.

3. Zusammensetzung, umfassend Melatonin, ein oder mehrere Carotenoide und Zink in einer zum Verbessern der kognitiven Funktion zum Verwenden beim Vermeiden, Reduzieren oder Verzögern eines Abbaus der kognitiven Funktionen in einem Haustier wirksamen Menge, wobei die Zusammensetzung dem Haustier in einer zum Verbessern der kognitiven Funktion des Haustiers wirksamen Menge verabreicht wird, wobei die Zusammensetzung von 0,1 bis 80 mg/kg Melatonin, von 0,01 bis 20 mg/kg von einem oder mehreren Carotenoiden und von 0,1 bis 400 mg/kg Zink umfasst, und wobei das Haustier aus einem Hund oder einer Katze ausgewählt ist.

4. Zusammensetzung zum Verwenden nach Anspruch 3, wobei das Melatonin ein synthetisches Melatonin ist.

5. Zusammensetzung zum Verwenden nach Anspruch 3, wobei die Carotenoide Xanthophylle sind; wahlweise wobei die Xanthophylle ausgewählt sind aus der Gruppe bestehend aus Astaxanthin, Lutein, Zeaxanthin, Neoxanthin, Violaxanthin, a-Cryptoxanthin und β-Cryptoxanthin; wobei das Xanthophyll wahlweise Astaxanthin ist.

6. Zusammensetzung zum Verwenden nach Anspruch 3, wobei der Hund oder die Katze ein alternder Hund oder eine alternde Katze ist.

7. Zusammensetzung zum Verwenden nach Anspruch 3, wobei der Hund oder die Katze für einen vom Alterungsprozess oder durch Verletzung oder Erkrankung hervorgerufenen Abbau der kognitiven Funktion anfälliger oder darunter leidender Hund oder anfällige oder darunter leidende Katze ist.

8. Zusammensetzung zum Verwenden nach Anspruch 3, wobei die Zusammensetzung dem Hund oder der Katze gemeinsam mit einem oder mehreren kognitiven Medikamenten in einer zum Verbessern der kognitiven Funktion wirksamen Menge verabreicht wird; wobei das kognitive Medikament wahlweise eins oder mehrere von Selegilin, Nicerogolin, einem Phosphatidylserin, Propentofyllin, Galantamin, Vinpocetin, Donepezil, einem *Ginkgo* biloba-Extrakt, einem Bisphosphonat, Raloxifen, einem Östrogen, einem Phytoöstrogen, Calcitonin, Risedronat oder Alendronat ist.

9. Zusammensetzung zum Verwenden nach Anspruch 3, wobei der Hund oder die Katze ein alternder Hund oder eine alternde Katze ist; wahlweise wobei der Hund oder die Katze einen mit altersbedingter kognitiver Beeinträchtigung assoziierten Phänotyp aufweist; wobei der Phänotyp wahlweise eines oder mehrere von verringerter Erinnerungsfähigkeit, Verlust des Kurzzeitgedächtnisses, verringerter Lernrate, verringerter Lernkapazität, verringerte Problemlösefähigkeiten, verringerter Aufmerksamkeitsspanne, vermehrter Verwirrung oder Demenz einschließt, verglichen mit einem Kontrollhund oder einer Kontrollkatze, der oder die diesen Phänotyp nicht aufweist.

10. Zusammensetzung, umfassend Melatonin, ein oder mehrere Carotenoide und Zink in einer zum Reduzieren oder Vermeiden altersbedingter Verhaltensänderungen zum Verwenden beim Vermeiden, Reduzieren oder Verzögern eines Abbaus der kognitiven Funktionen in einem Hund oder einer Katze wirksamen Menge, wobei die Zusammensetzung dem Hund oder der Katze in einer zum Reduzieren oder Vermeiden altersbedingter Verhaltensänderungen im Hund oder in der Katze wirksamen Menge verabreicht wird, wobei die Zusammensetzung von 0,1 bis 80 mg/kg Melatonin, von 0,01 bis 20 mg/kg von einem oder mehreren Carotenoiden und von 0,1 bis 400 mg/kg Zink umfasst,

## Revendications

1. Composition appropriée pour améliorer la fonction cognitive chez un animal de compagnie, comprenant de la mélatonine et un ou plusieurs caroténoïdes et du zinc en une quantité efficace pour améliorer la fonction cognitive chez l'animal de compagnie, où la composition comprend de 0,1 à 80 mg/kg de mélatonine, de 0,01 à 20 mg/kg d'un ou plus de caroténoïdes.

2. Composition selon la revendication 1, comprenant d'environ 0,1 à 400 mg/kg de zinc.

3. Composition comprenant de la mélatonine, un ou plusieurs caroténoïdes et du zinc en une quantité efficace pour améliorer la fonction cognitive à utiliser pour prévenir, réduire ou retarder un déclin des fonctions cognitives chez un animal de compagnie, où la composition est destinée à être administrée à l'animal de compagnie en une quantité efficace pour améliorer la fonction cognitive de l'animal de compagnie, où la composition comprend de 0,1 à 80 mg/kg de mélatonine, de 0,01 à 20 mg/kg d'un ou de plusieurs caroténoïdes et de 0,1 à 400 mg/kg de zinc et dans laquelle l'animal de compagnie est choisi parmi un animal canin ou félin.

4. Composition à utiliser selon la revendication 3, dans laquelle la mélatonine est une mélatonine synthétique.

5. Composition à utiliser selon la revendication 3, dans laquelle les caroténoïdes sont des xanthophylles ; facultativement dans laquelle les xanthophylles sont choisies parmi le groupe composé d'astacanthine, de lutéine, de zéaxanthine, de néoxanthine, de violaxanthine, d'a-cryptoxanthine et de β-cryptoxanthine ; facultativement dans laquelle la xanthophylle est l'astaxanthine.

6. Composition à utiliser selon la revendication 3, dans laquelle l'animal canin ou félin est un animal canin ou félin vieillissant.

7. Composition à utiliser selon la revendication 3, dans laquelle l'animal canin ou félin est un animal canin ou félin susceptible ou souffrant d'un déclin de la fonction cognitive entraîné par le processus de vieillissement ou par blessure ou maladie.

8. Composition utilisable selon la revendication 3, où la composition est administrée à l'animal canin ou félin conjointement avec un ou plusieurs médicaments cognitifs en une quantité efficace pour améliorer la fonction cognitive ; facultativement dans laquelle le médicament cognitif est un médicament ou plusieurs parmi sélégiline, nicérogoline, phosphatidylsérine, propentofyline, galantamine, vinpocétine, donépézil, un extrait de *Ginkgo biloba,* un bisphosphonate, le raloxifène, un oestrogène, un phyto-oestrogène, la calcitonine, le risédronate ou l'alendronate.

9. Composition à utiliser selon la revendication 3, dans laquelle l'animal canin ou félin est un animal canin ou félin vieillissant ; facultativement dans laquelle l'animal canin ou félin présente un phénotype associé à des troubles cognitifs liés à l'âge ; facultativement dans laquelle le phénotype comprend un ou plusieurs éléments parmi une capacité affaiblie de mémoire, une perte de mémoire à court terme, une vitesse d'apprentissage réduite, une capacité affaiblie d'apprentissage, la diminution des capacités à résoudre les problèmes, une capacité de concentration diminuée, une confusion accrue ou la démence, en comparaison avec un animal canin ou félin témoin ne présentant pas le phénotype.

10. Composition comprenant de la mélatonine, un ou plusieurs caroténoïdes et du zinc en une quantité efficace pour réduire ou prévenir des changements de comportements liés à l'âge à utiliser pour prévenir, réduire ou retarder un déclin des fonctions cognitives chez un animal canin ou félin, dans laquelle la composition est administrée à l'animal canin ou félin en une quantité efficace pour réduire ou prévenir les changements de comportements liés à l'âge de l'animal canin ou félin, dans laquelle la composition comprend de 0,1 à 80 mg/kg de mélatonine, de 0,01 à 20 mg/kg d'un ou de plusieurs caroténoïdes et de 0,1 à 400 mg/kg de zinc.
